# EUROPÄISCHE PATENTSCHRIFT

(11) **EP 2 275 763 B1**
(45) Veröffentlichungstag und Bekanntmachung des Hinweises auf die Patenterteilung: **04.03.2015**
(21) Anmeldenummer: 09009127.3
(22) Anmeldetag: 13.07.2009
(51) Int. Cl.: F26B 23/10, C12M 1/00, F26B 9/10

(54) **Vorrichtung zur Behandlung von Material, insbesondere von Gärresten oder Gärrestmischungen**
Device for treating materials, especially fermentation residues and mixtures of fermentation residues
Dispositif de traitement de matériaux, notamment de résidus de fermentation ou de mélanges de résidus de fermentation

(43) Veröffentlichungstag der Anmeldung: 19.01.2011
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A- 1 428 868
- EP-A2- 1 564 515
- WO-A-2008/095685
- DE-A1- 2 332 363
- DE-A1- 2 942 325
- DE-A1- 3 513 159
- DE-A1- 4 314 645
- DE-A1- 19 609 530
- DE-A1- 19 851 793
- DE-A1-102004 042 285
- DE-A1-102007 038 105
- DE-B- 1 161 217
- DE-B3-102005 005 859
- DE-C- 492 226
- DE-U1-202006 002 757

## Beschreibung

Die Erfindung betrifft eine Vorrichtung zur Behandlung von Material, insbesondere von Gärresten oder Gärrestmischungen, nach dem Oberbegriff des Anspruchs 1.

Obwohl im Folgenden Gärrest als Material erwähnt wird, bezieht sich die Erfindung auch auf Mischungen aus Gärrest und anderen Materialien sowie ganz allgemein auf die Behandlung von Materialien, die zur Aufbereitung durch Wärmezufuhr zu behandeln sind.

Entsprechende Einrichtungen werden zum Beispiel verwendet, um einen aus einem Prozess, z.B. einem Fermentationsprozess, entnommenen Gärrest oder ein Gemisch aus Gärresten und anderen Materialien nachzubehandeln. Bei der Nachbehandlung wird der Gärrest, der mit einer Temperatur von ca. 35°C aus dem Prozess kommt, für eine bestimmte Zeit auf einem höheren Temperaturniveau nachbehandelt. Dabei weist der Gärrest in der Regel eine große Menge an Flüssigkeit, insbesondere Wasser, auf, welche bei über 50% liegt, so dass der Gärrest weiter zu bearbeiten ist, damit der Wasseranteil deutlich sinken kann, so dass insbesondere eine Kompostierung möglich wird.

Zur erforderlichen Erwärmung wird oftmals ein in einer Leitung erwärmter Luftstrom durch eine Bodenplatte eines Behandlungsbehälters in den Bereich des Gärrestes eingeblasen. Durch diese Maßnahme soll eine möglichst homogene Verteilung des erwärmten Mediums Luft im Gärrest erreicht werden, damit dieser möglichst durch die Luft erwärmt wird. Aufgrund der geringen spezifischen Wärmekapazität von Luft ist dieser Prozess sehr langwierig, da sich die Luft, die sich beim Durchdringen des Gärrestes abkühlt, als Abluft aufgefangen und wieder erwärmt werden muss, bevor sie wieder in den Einlass des Behandlungsbehälters eingeblasen werden kann. Die Erwärmung des Gärrestes erfordert bei dieser Vorgehensweise eine große Anzahl von Zyklen. Das bedeutet aber auch, dass für das Erwärmen der Luft eine große Energiemenge erforderlich ist, welche mit der Anzahl der erforderlichen Zyklen wächst.

Der Erfindung liegt daher die Aufgabe zugrunde, eine Vorrichtung der eingangs genannten Art anzugeben, welche wesentlich effektiver im Hinblick auf den erforderlichen Behandlungszeitraum und die zugehörige Energiebilanz arbeitet.

Gelöst wird diese Aufgabe mit den Merkmalen des Anspruchs 1. Vorteilhafte Ausführungsformen finden sich in den abhängigen Ansprüchen.

Erfindungsgemäß ist im Boden der erfindungsgemäßen Vorrichtung, welcher Teil eines Behandlungsbehälters sein kann, eine Heizeinrichtung vorgesehen. So steht für zu behandelndes Material, insbesondere Gärrest, eine wenigstens durch den Boden gebildete erwärmte Fläche zur Verfügung, die das Material "von unten" mit deutlich verringertem Energieaufwand erwärmt. Somit wird die Erwärmung des Materials wesentlich effektiver. Bei geschlossenen Behältern sind zudem wesentlich weniger Zyklen für die Wiedererwärmung der aus dem Behälter kommenden Abluft erforderlich als bei der herkömmlichen Verfahrensweise.

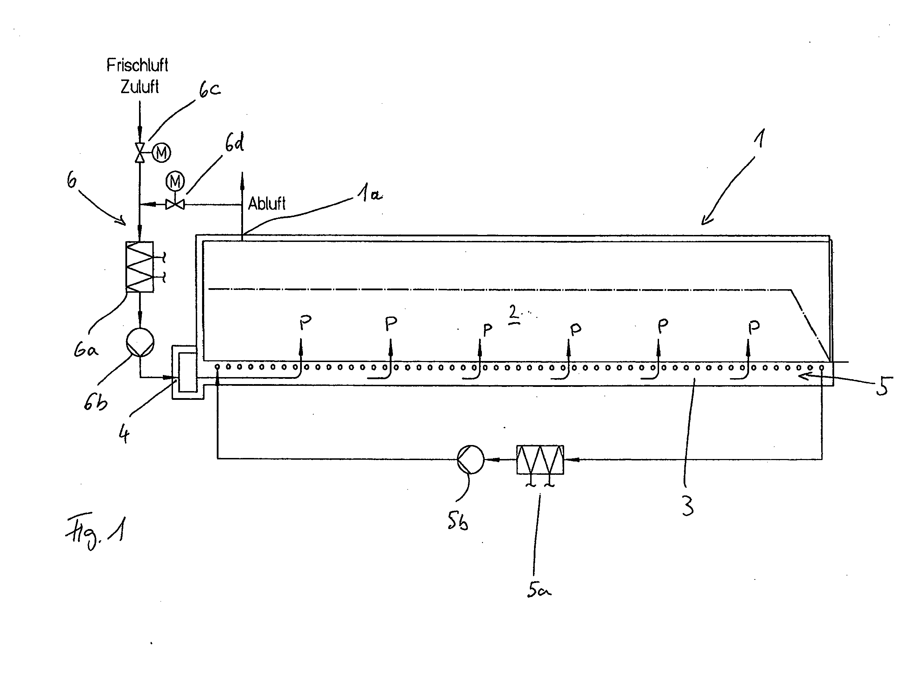

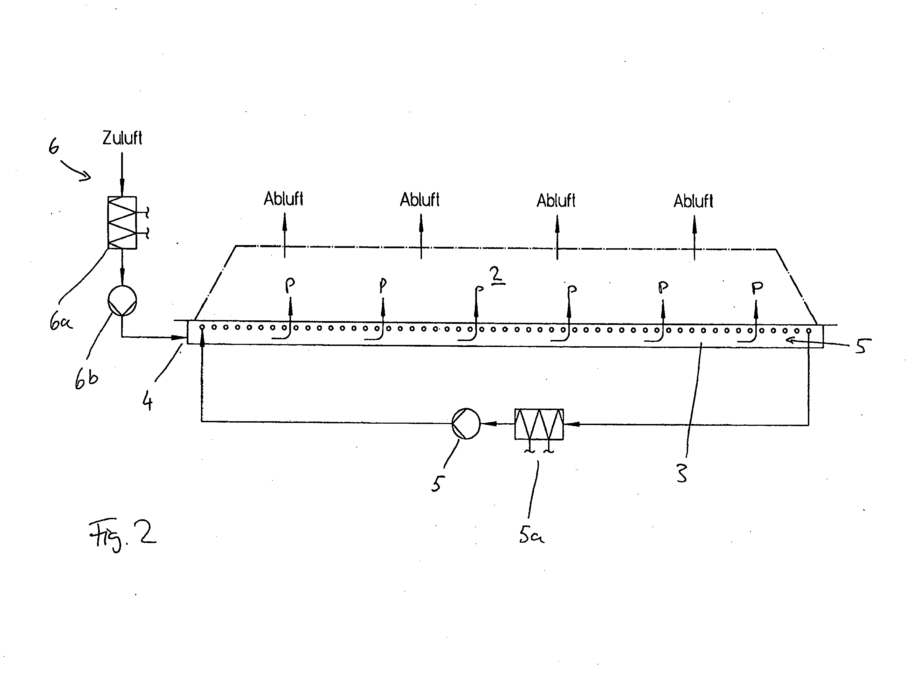

Die Erfindung wird nun anhand der in den Figuren 1 und 2 gezeigten Ausführungsbeispiele schematisch näher erläutert, wobei beispielhaft das zu behandelnde Material im Folgenden als Gärrest bezeichnet wird.
- Figur 1 -: zeigt eine erste Ausführungsform der erfindungsgemäßen Vorrichtung in Form eines Rottebehälters,
- Figur 2 -: zeigt eine zweite Ausführungsform der erfindungsgemäßen Vorrichtung in Form einer Miete.

Die in Figur 1 gezeigte Vorrichtung weist einen Behälter 1 auf, in welchem der Gärrest oder ein Gemisch mit Gärresten 2 oder auch anderes nachzubehandelndes Material auf dem Behälterboden 3 angeordnet wird, nachdem der Gärrest 2 etwa aus einem Fermenter entnommen worden ist. Der Boden 3 ist gasdurchlässig ausgebildet, so dass ein gasförmiges Medium (im Folgenden Luft) über einen oder mehrere Einlässe 4 über ein Gebläse 6b aus einer Leitung 6 in den Behälter unter Druck eingeblasen werden kann. Die in den Behälter eingeblasene Luft kann als Frischluft über ein Ventil 6c zugeführt werden oder/und über einen oder mehrere Auslässe 1a aus dem Behälter 1 als Abluft entnommen werden. Die Regelung der zuzuführenden Ströme erfolgt über die Ventile 6c und 6d. Abhängig von der Temperatur der Luft kann eine Heizung 6a, die in die Leitung 6 integriert ist oder nach Art eines Wärmetauschers mit der Leitung 6 zusammenwirkt, für die Temperatureinstellung sorgen.

Der Gärrest weist bei der Entnahme aus einem Fermentationsprozess eine Temperatur von ca. 35°C auf, die zur Behandlung auf ein deutlich höheres Temperaturniveau anzuheben ist. Die Anhebung erfolgt zum einen mit dem über die Leitung 6 zugeführten Gas bzw. der so zugeführten Luft, die durch den durchlässigen Boden 3 in den Behälter 1 gelangt und dadurch mit Druck im Gärrest verteilt wird. Zum anderen ist im Boden in direkter Nachbarschaft zum Gärrest 2 eine Heizeinrichtung 5 vorgesehen, welche bevorzugt im Boden 3 vorgesehen ist und mittels einer Pumpe 5b mit einem über die Heizung 5a beheizten Heizmittel durchströmt wird. Hierdurch wird das zu behandelnde Substrat infolge direkter Wärmeleitung erwärmt. Die zugeführte Luft gelangt - wie durch die Pfeile P angedeutet - in den Behälter und verteilt sich im Gärrest 2, durchströmt und erwärmt diesen ebenfalls und kann als dann abgekühlte Abluft über den Auslass 1a wieder rezykliert werden. So wird ein Wärmeverlust der Luft durch lange Transportwege vermieden und der Energieaufwand reduziert. Dadurch kann bei gleichem Energieeinsatz eine wesentlich bessere Erwärmung des Gärrestes 2 erfolgen.

In Figur 2 ist eine zweite erfindungsgemäße Ausführungsform gezeigt, bei der die Nachbehandlung des Gärrestes 2 nicht in einem geschlossenen Behälter (vgl. Figur 1), sondern offen stattfindet. Hierzu ist lediglich der oben bereits beschriebene, in Richtung des Gärrestes durchlässige Boden 3 vorgesehen, in welchen über den Einlass 4 die aus der Leitung 6 zugeführte Zuluft bzw. ein anderes gasförmiges Medium eingebracht wird. Der Heizvorgang erfolgt wie beim obigen Ausführungsbeispiel, so dass entsprechend auf die obigen Ausführungen verwiesen wird. Die aus dem Gärrest 2 austretende Abluft wird in diesem Fall nicht rezykliert, sondern gelangt in die Atmosphäre.

## Patentansprüche

1. Vorrichtung zur Behandlung von Gärresten oder Gärrestmischungen (2), aufweisend einen Boden (3) zur Ablage des Gärresten (2), wobei der Boden (3) gasdurchlässig ausgebildet ist, und wobei der Boden (3) eine an eine Leitung (6) anschließbare Einlasseinrichtung (4) zum Einbringen eines gasförmigen Mediums in den Boden (3) aufweist, die so ausgelegt ist, dass wenigstens Teile des Bereichs des Bodens (3) zum Belüften des Gärrestes (2) von dem gasförmigen Medium durchströmt werden können, wobei in die Leitung (6) eine Heizeinrichtung (6a) zum Heizen des durch die Leitung (6) strömenden gasförmigen Mediums geschaltet ist,
**dadurch gekennzeichnet,**
**dass** im Boden (3) wenigstens ein Heizelement (5) vorgesehen ist, wodurch der Gärrest (2) infolge direkter Wärmeeinleitung erwärmt wird, wobei die Leitung (6) zum Zuführen von Zuluft ausgebildet ist.

2. Vorrichtung nach Anspruch 1,
**dadurch gekennzeichnet**
**dass** der Boden (3) Teil eines Behälters (1) zur Aufnahme des Gärrestes oder Gärrestgemisches (2) ist.

3. Vorrichtung nach Anspruch 2,
**dadurch gekennzeichnet,**
**dass** der Behälter (1) wenigstens eine Auslasseinrichtung (1a) zum Auslassen eines im Behälter (1) befindlichen gasförmigen Mediums aufweist.

4. Vorrichtung nach einem der vorherigen Ansprüche,
**dadurch gekennzeichnet,**
**dass** die Einrichtung (4) zum Einbringen des gasförmigen Mediums mit einer Leitung (6) verbunden ist.

5. Vorrichtung nach einem der Ansprüche 2 oder 3 und Anspruch 4,
**dadurch gekennzeichnet,**
**dass** die Auslasseinrichtung (1a) und die Einlasseinrichtung (4) miteinander zur Rezyklierung des gasförmigen Mediums über die Leitung (6) verbunden oder verbindbar sind.

6. Vorrichtung nach Anspruch 5,
**dadurch gekennzeichnet,**
**dass** die Leitung (6) so geschaltet ist, dass sie alternativ oder ergänzend zu dem aus der Auslasseinrichtung (1a) kommenden gasförmigen Medium von außerhalb des Behälters (1) mit Frischluft gespeist werden kann.

7. Verfahren zum Betrieb einer Vorrichtung zur Behandlung von Gärresten oder Gärrestmischungen (2), insbesondere nach einem der vorherigen Ansprüche, bei dem ein Gärrest oder Gärrestgemisch nach deren Entnahme aus einem Fermenter in einen geschlossenen Behälter (1) eingebracht und anschließend mit einem erwärmten Gasförmigen Medium durchströmt wird,
**dadurch gekennzeichnet,**
**dass** die Erwärmung des Gärrests oder Gärrestgemisches (2) wenigstens teilweise durch Erwärmen des Bodens (3) des Behälters (1) mittels eines beheizten Heizmittels erfolgt, wobei das Heizmittel von dem gasförmigen Medium verschieden ist.

## Claims

1. Device for treating fermentation residues or mixtures (2) of fermentation residues, having a base (3) for supporting the fermentation residue (2), wherein the base (3) is designed to be gas-permeable and wherein the base (3) has an inlet device (4) connectable to a pipeline (6) for introducing a gaseous medium into the base (3), which is designed so that at least parts of the regions of the base (3) are permeated by the gaseous medium for aerating the fermentation residue (2), wherein a heating device (6a) is switched into the pipeline (6) for heating the gaseous medium flowing through the pipeline (6),
**characterised in that**
in the base (3) there is at least one heating element (5) whereby the fermentation residue (2) is heated up as a result of direct heat conduction, wherein the pipeline (6) is designed for supplying supply air.

2. Device according to claim 1
**characterised in that**
the base (3) is a part of a container (1) for receiving the fermentation residue or mixture of fermentation residues (2).

3. Device according to claim 2
**characterised in that**
the container (1) has at least one outlet device (1a) for the removal of a gaseous medium located in the container (1).

4. Device according to one of the preceding claims
**characterised in that**
the device (4) for introducing the gaseous medium is connected to a pipeline (6).

5. Device according to one of claims 2 or 3 and claim 4
**characterised in that**
the outlet device (1a) and the inlet device (4) are connected or connectable to one another for recycling the gaseous medium via the pipeline (6).

6. Device according to claim 5
**characterised in that**
the pipeline (6) is switched in so that it can be fed with fresh air from outside the container (1) alternatively or additionally to the gaseous medium coming from the outlet device (1a).

7. Method for operating a device for the treatment of fermentation residues or mixtures of fermentation residues (2), more particularly according to one of the preceding claims, in which a fermentation residue or a mixture of fermentation residues after its removal from a fermenter is introduced into a closed container (1) and is then permeated by a heated gaseous medium,
**characterised in that**
the heating of the fermentation residue or mixture of fermentation residues (2) takes place at least partially by heating up the base (3) of the container (1) by means of a heated heating medium, wherein the heating medium is different from the gaseous medium.

## Revendications

1. Dispositif de traitement de résidus de fermentation ou de mélanges de résidus de fermentation (2), qui présente un fond (3) pour le dépôt des résidus de fermentation (2), sachant que le fond (3) est de conception perméable aux gaz, et sachant que le fond (3) est doté d'un système d'admission (4) qui, servant à l'introduction d'un fluide gazeux dans ledit fond (3), peut être raccordé à une conduite (6), ledit système étant conçu de telle manière qu'au moins les parties de la région du fond (3) puissent être traversées par le fluide gazeux pour aérer les résidus de fermentation (2), sachant que, dans la conduite (6), est branché un dispositif de chauffage (6a), qui est destiné à chauffer le fluide gazeux s'écoulant à travers ladite conduite (6),
**caractérisé en ce que**,
dans le fond (3), est prévu au moins un élément de chauffage (5), grâce auquel les résidus de fermentation (2) sont chauffés par apport de chaleur direct, sachant que la conduite (6) est conçue pour l'amenée d'air d'alimentation.

2. Dispositif selon la revendication 1,
**caractérisé en ce que**
le fond (3) fait partie d'un conteneur (1) qui est destiné à recevoir des résidus de fermentation ou des mélanges de résidus de fermentation (2).

3. Dispositif selon la revendication 2,
**caractérisé en ce que**
le conteneur (1) présente au moins un dispositif d'évacuation (1a) pour l'évacuation d'un fluide gazeux se trouvant dans ledit conteneur (1).

4. Dispositif selon l'une des revendications précédentes, **caractérisé en ce que**
le système d'admission (4) du fluide gazeux est relié à une conduite (6).

5. Dispositif selon l'une des revendications 2 ou 3 et revendication 4,
**caractérisé en ce que**
le dispositif d'évacuation (1a) et le système d'admission (4) peuvent être reliés ensemble par l'intermédiaire de la conduite (6) pour le recyclage du fluide gazeux.

6. Dispositif selon la revendication 5,
**caractérisé en ce que**
la conduite (6) est branchée de telle manière qu'elle puisse être alimentée en air frais, à partir de l'extérieur du conteneur (1), soit alternativement ou complémentairement, en plus du fluide gazeux en provenant du dispositif d'évacuation (1a).

7. Procédé d'exploitation d'un dispositif de traitement de résidus de fermentation ou de mélanges de résidus de fermentation (2), en particulier selon l'une des revendications précédentes, dans lequel un résidu de fermentation ou un mélange de résidus de fermentation, après avoir été prélevés d'un fermenteur, sont amenés dans un conteneur (1) fermé et traversés ensuite par un fluide gazeux, chauffé,
**caractérisé en ce que**
le chauffage du résidu de fermentation ou du mélange de résidus de fermentation (2) est effectué, au moins partiellement, par chauffage du fond (3) du conteneur (1) au moyen d'un agent de chauffage chauffé, sachant que l'agent de chauffage est différent du fluide gazeux.
